# EUROPEAN PATENT SPECIFICATION

(11) **EP 0 372 889 B1**
(45) Date of publication and mention of the grant of the patent: **24.03.1993**
(21) Application number: 89312626.8
(22) Date of filing: 04.12.1989
(51) Int. Cl.: A61K 31/71, A61K 47/08, A61K 47/10, A61K 47/12

(54) **Doxorubicin solutions**
Doxorubicin-Lösungen
Solutions de doxorubicine

(30) Priority: 05.12.1988 US 279626
(43) Date of publication of application: 13.06.1990
(73) Proprietor: Bristol-Myers Squibb Company, New York, N.Y. 10154 (US)
(72) Inventor: Kaplan, Murray A., Syracuse, NY 13206 (US); Perrone, Robert K., Liverpool, NY 13090 (US); Bogardus, Joseph B., Manlius, NY 13104 (US)
(74) Representative: Jones, Alan John

(56) References cited:
- EP-A- 0 299 528
- GB-A- 2 178 311

## Description

The present invention relates to stable concentrated solutions of doxorubicin which may be diluted with sterile water or other suitable diluent for parenteral administration.

Doxorubicin is an antineoplastic antibiotic which is isolated from the fermentation of Streptomyces peucetius var. caesius. It can also be synthesized chemically from daunorubicin.

The commercial lyophilized dosage form of doxorubicin (marketed by Adria Laboratories as Adriamycin®) is supplied as the hydrochloride salt in 10, 20 and 50 mg vials in combination with lactose at 50, 100 and 250 mg, respectively. Vials are reconstituted with Sodium Chloride Injection, USP (0.9%) or Sterile Water for Injection, USP, to give a final concentration of 2 mg/ml of doxorubicin HCl and a pH range of 4-6. The reconstituted lyophilized solution is reported by the manufacturer to be stable (≦ 10% potency loss) for only 24 hours at room temperature and 48 hours under refrigeration.

It would be desirable to have a solution dosage form of doxorubicin which would not require reconstitution prior to use. Improper reconstitution of a lyophilized product sometimes results in the formation of air-borne droplets ("blow-back") which, in the case of a potent antitumor agent such as doxorubicin, may be a health hazard to the personnel making up the solution for injection. Also, production of the present lyophilized doxorubicin HCl dosage form is quite costly, and a solution dosage form would be expected to have a lower cost of goods.

Doxorubicin HCl has recently been made commercially available as a preservative-free aqueous solution. Despite the convenience of a ready-to-use solution, the marketed aqueous solution is still not as stable as one would desire. The manufacturer indicates that the ready-to-use solution is stable for 18 months at 4°C. At the end of this time period, the solution may contain up to 10% of undesirable and potentially toxic degradation products.

A study carried out by Hoffman, et al. and reported in Am. J. Hosp. Pharm. 36: 1536-1538, 1979 indicates that reconstituted solutions of doxorubicin HCl are stable (≦10% potency loss) for up to six months when refrigerated at 4°C. Ketcyhum, et al. in Am. J. Intrav. Ther. Clin. Nutri. 8: 15-18, 1981, state that a reconstituted solution of doxorubicin HCl in 0.9% NaCl solution remains stable at room temperature or 5°C for seven days. Despite such indications that reconstituted doxorubicin HCl may be sufficiently stable for up to six months at 4°C, this is still insufficient for a commercial solution dosage form.

Janssen, et al. in Int. J. Pharmaceutics 23: 1-11, 1985, report that doxorubicin HCl appears to have its maximum stability at pH 4(Tris or phosphate buffers using HCl to adjust pH). Poochikian, et al. in Am. J. Hosp. Pharm. 38: 483-486, 1981, note that the stability of doxorubicin HCl is pH dependent and that the optimum stability is achieved when the reconstitution solvent is 5% Dextrose Injection, USP, having a pH of approximately 4.5.

GB-A- 2,178,311 discloses solutions of doxorubicin HCl in aqueous and non-aqueous solvent systems. There is no disclosure, however, of use of the antioxidant, sodium acetone bisulfite, to stabilize such solutions.

EP-A- 273,603 discloses solutions of doxorubicin HCl in aqueous and non-aqueous solvent systems and appears to be substantially equivalent to GB-A- 2,178,311 except for limitation of the pH to the range of 2.5 to 4.0 instead of the broader pH range of 2.5 to 6.5 disclosed in UK 2,178,311.

GB-A- 4,675,311 discloses aqueous solutions of doxorubicin HCl containing a co-solubilizing agent selected from the group comprising a hydroxy-, mercapto-, or amino-substituted benzoic acid, an alkali metal salt thereof, a C₁-C₄ alkyl ester thereof, a ring-halogenated methyl-substituted phenol, an amino acid, and mixtures thereof.

Applicants have previously filed EP-A-0299528, and co-pending U.S. application Serial Number 246,602 on September 20, 1988, which disclose and claim stable, ready-to-use non-aqueous solutions of doxorubicin HCl containing doxorubicin HCl, citric acid and an antioxidant selected from sodium formaldehyde bisulfite, tert-butylhydroquinone and α-tocopherol in a solvent medium consisting of propylen glycol, 1,2-dihydroxybutane or 1,3-dihydroxypropane, or mixtures of such non-aqueous solvents with water.

The antioxidant, sodium acetone bisulfite, used in the present invention has been previously utilized as a stabilizing agent for other pharmaceutical products. For example, Ca-A- 981,182 discloses use of sodium acetone bisulfite in combination with thioglycerol and ascorbic acid to stabilize γ-epinephrine aqueous solutions. Sodium acetone bisulfite is also used as a preservative in other pharmaceutical solutions, e.g. pentazocine lactate and procaine hydrochloride.

It is an object of the present invention to provide a non-aqueous doxorubicin solution dosage form which is stable (≦10% potency loss) for at least 18 months at room temperature and at least four months at 37°C, which contains at least about 1 mg of doxorubicin per ml of solution and which can be diluted with water or other aqueous vehicle for parenteral administration.

### SUMMARY OF THE INVENTION

The present invention provides a stable, sterile, doxorubicin solution in a sealed container suitable for dilution with sterile water or other aqueous vehicle for parenteral administration, said solution containing a physiologically acceptable salt of doxorubicin, a stabilizing amount of sodium acetone bisulfite of 0.1 to 20 mg/ml and, optionally, an amount of a physiologically acceptable acid or base necessary to provide a solution pH in the range of 3-6, in a solvent medium consisting of 90-100% (v/v) of propylene glycol, 1,2-dihydroxybutane or 1,3-dihydroxypropane and 0-10% (v/v) water.

### DETAILED DESCRIPTION

This invention relates to stable, concentrated, solutions of a physiologically acceptable salt of doxorubicin suitable, upon dilution with sterile water or other suitable aqueous vehicle, for parenteral administration. Depending on the solvent medium employed, concentrations of from 1 to 20 mg/ml doxorubicin salt are used in the solutions. More particularly, concentrations of from 1 to 10 mg/ml are utilized with neat propylene glycol, 1,2-dihydroxybutane or 1,3-dihydroxypropane, and concentrations of from 1 to 20 mg/ml are used when 5-10% (v/v) water is mixed with the above-mentioned propylene glycol, 1,2-dihydroxybutane or 1,3-dihydroxypropane solvents.

Although the present invention is generally described below in terms of doxorubicin hydrochloride, any physiologically acceptable salt of doxorubicin may be used for preparing the solutions. Examples of suitable salts include the salts with mineral inorganic acids such as hydrochloric, hydrobromic, sulfuric-phosphoric and nitric, and the salts with organic acids such as acetic, succinic, tartaric, ascorbic, citric, glutamic, benzoic, methanesulfonic, and 2-hydroxyethylsulfonic (isethionic acid). The preferred physiologically acceptable salt is doxorubicin hydrochloride.

Unless indicated otherwise the term "non-aqueous" as used herein refers to a solution which contains up to about 10% (v/v) water.

Doxorubicin HCl at room temperature has a solubility of less than 5 mg/ml in propylene glycol, 1,2-dihydroxybutane and 1,3-dihydroxypropane. Upon short-term (0.5-1 hour) warming (60-80°C) with vigorous stirring, however, solubility of up to about 10 mg/ml can be achieved with no potency loss. The doxorubicin HCl remains in solution when stored at 4°C to -20°C and seeding of the solutions with crystalline doxorubicin HCl does not affect crystallization. Solubility of doxorubicin HCl can be increased to about 20 mg/ml in the above-mentioned solvents by addition of 5-10% (v/v) water.

Solubilities of doxorubicin HCl in other solvents such as polyethylene glycols, ethanol, benzyl alcohol, dimethylisosorbide, ethyl lactate, Solketol, Cremophors, and Pluronics were less than 5 mg/ml with heating and thus these solvents did not meet our minimum 5 mg/ml criterion for a solution form of doxorubicin. Other solvents tested such as glycerol formal, 2-pyrrolidinone and 1-methyl-2-pyrrolidinone produced solutions of 5 mg/ml or greater but were chemically unstable.

One critical component of doxorubicin non-aqueous solutions is pH with a pH range of 3-6 being necessary for acceptable long-term stability. The pH is measured on solutions diluted to 1 mg/ml with water or 70% water/30% acetonitrile (v/v). If pH adjustment is necessary to achieve the required pH 3-6 range, common physiologically acceptable acids and bases can be added. For example, the solution pH can be lowered by addition of suitable organic or inorganic acids such as hydrochloric, hydrobromic, sulfuric, phosphoric, nitric, acetic, succinic, tartaric, ascorbic, citric, glutamic, benzoic, methanesulfonic, ethanesulfonic, or 2-hydroxyethylsulfonic acid, Solution pH may be raised by addition of physiologically acceptable bases such as sodium carbonate, sodium bicarbonate, or sodium hydroxide. A particularly preferred acid for pH control is citric acid since this reagent acts as a chelation agent as well as providing pH control.

It is also necessary for optimum stability of the solutions, especially at elevated temperatures which may be encountered at some time during the life of the product, e.g. during shipping, to utilize an antioxidant. We have tested a number of antioxidants including α-tocopherol, tert-butylhydroquinone (TBHQ), sodium formaldehyde bisulfite, butylated hydroxy anisole (BHA), butylated hydroxy toluene (BHT), sodium bisulfite and propyl gallate. Of these only α-tocopherol, tert-butylhydroquinone and sodium formaldehyde bisulfite resulted in the desired stability at elevated temperatures as well as at room temperature (25°C) or below. This finding was disclosed and claimed in our EP-A-0299528 and in co-pending U.S. application Serial Number 246,602 filed September 20, 1988. Later work found that another antioxidant, sodium acetone bisulfite, gave even superior stabilizing properties to doxorubicin solutions, and it is this discovery which is the subject of the present invention. Generally, an amount of sodium acetone bisulfite is employed in the range of 0.1 to 20 mg/ml with 2 mg/ml being most preferred.

Despite reports of potency losses of doxorubicin HCl solutions via photolytic degradation and adsorption to container walls, we have not noted any significant loss of potency due to container adsorption or sensitivity to normal room light with our solutions. Glass vials may conveniently be used as the container.

We have also found that deaeration of the solvent medium is preferred. This is accomplished by heating to 100°C and/or vacuum prior to formulation, and purging with an inert gas such as nitrogen, helium or argon. Deaeration of the filled final formulation vials before they are sealed contributes to optimum stability and is preferred. In contrast to the antioxidants previously tested, including α-tocopherol, tert-butylhydroquinone and sodium formaldehyde bisulfite, inert gas purging was found to be less necessary when sodium acetone bisulfite is employed. This is another indication of the unusual effectiveness of this particular antioxidant for stabilization of doxorubicin solutions. Because of this property, manufacturing control will be simplified since air need not be totally eliminated.

As an example of a stable non-aqueous solution of doxorubicin, 20 mg/ml of doxorubicin hydrochloride, 0.2 mg/ml citric acid (anhydrous), 2.0 mg/ml of sodium acetone bisulfite and a solvent medium consisting of deaerated propylene glycol/water (90:10 v/v) are added to a Type 1 flint vial, the vial is purged with nitrogen and then sealed with a suitable teflon-coated stopper. This solution retained 97.2% potency after 2 weeks at 40°C, 93.4% after 4 weeks at 40°C and 88.2% after 12 weeks at 40°C and would have an acceptable shelf-life (≦10% potency loss) of at least 18 months at room temperature and at least four months at 37°C. Substantially equivalent results are obtained when 1,2-dihydroxybutane and 1,3-dihydroxypropane solvent systems are used.

The solutions provided by the present invention may be diluted with sterile water or other conventional sterile aqueous vehicle (e.g. saline solution) to a concentration of 2 mg/ml or less and are then employed for the treatment of cancer in the same manner as the present commercial aqueous solution dosage form or reconstituted lyophilized form. They may also be used without dilution in infusion pump systems for long term i.v. infusion.

## Claims (Claims for the following Contracting State(s): AT, BE, CH, DE, FR, GB, IT, LI, LU, NL, SE)

1. A pharmaceutical formulation of a stable, sterile, non-aqueous solution of doxorubicin in a sealed container, said solution containing from 1 to 20 mg/ml of a physiologically acceptable salt of doxorubicin and 0.1 to 20 mg/ml of sodium acetone bisulfite in a solvent medium consisting of 90-100% (v/v) propylene glycol, 1,2-dihydroxybutane or 1,3-dihydroxypropane and 0-10% (v/v) water, and, if required, an amount of a physiologically acceptable acid or base necessary to adjust the solution pH to within the range of 3-6.

2. A formulation according to Claim 1 wherein a deaerated solvent medium is employed and the solution is subjected to inert gas purging before the container is sealed.

3. A formulation according to Claim 1 or 2 wherein the solvent medium is neat propylene glycol.

4. A formulation according to Claim 1 or 2 wherein the solvent medium is neat 1,2-dihydroxybutane.

5. A formulation according to Claim 1 or 2 wherein the solvent medium is neat 1,3-dihydroxypropane.

6. A formulation according to Claim 1 or 2 wherein the solvent medium is propylene glycol/water (90:10 v/v).

7. A formulation according to claim 1 or 2 wherein the solvent medium is propylene glycol/water (95:5 v/v).

8. A formulation according to claim 1 or 2 wherein the solvent medium is 1,2-dihydroxybutane/water (90-95:5-10 v/v).

9. A formulation according to any one of claims 1 to 8 wherein the solvent medium consists of 90-95% (v/v) propylene glycol, 1,2-dihydroxybutane or 1,3-dihydroxypropane and 5-10% (v/v) water.

10. A formulation according to claim 9 wherein the solvent medium is 1,3-dihydroxypropane/water (90-95:5-10 v/v).

11. A method of preparing a pharmaceutical formulation of a stable, sterile solution of doxorubicin, said method comprising the steps of mixing from 1 to 20 mg/ml of a physiologically acceptable salt of doxorubicin and 0.1 to 20 mg/ml of sodium acetone bisulfite with a solvent medium consisting of 90-100% (v/v) propylene glycol, 1,2-dihydroxybutane or 1,3-dihydroxypropane and 0-10% (v/v) water to form a solution, adjusting, if necessary, the pH of said solution to within the range of 3-6 by adding a physiologically acceptable acid or base, adding such solution to a container, and sealing said container.

12. A method of preparing a pharmaceutical formulation according to claim 11 wherein the amount of water is 5-10% (v/v).

13. A method according to claim 11 or claim 12 wherein a deaerated solvent medium is employed and the solution is subjected to inert gas purging before the container is sealed.

## Claims (Claims for the following Contracting State(s): ES, GR)

1. A method of preparing a pharmaceutical formulation of a stable, sterile, solution of doxorubicin, said method comprising the steps of mixing from 1 to 20 mg/ml of a physiologically acceptable salt of doxorubicin and 0.1 to 20 mg/ml of sodium acetone bisulfite with a solvent medium consisting of 90-100% (v/v) propylene glycol, 1,2-dihydroxybutane or 1,3-dihydroxypropane and 0-10% (v/v) water to form a solution, adjusting, if necessary, the pH of said solution to within the range of 3-6 by adding a physiologically acceptable acid or base, adding such solution to a container, and sealing said container.

2. A method of preparing a pharmaceutical formulation according to claim 1 wherein the amount of water is 5-10% (v/v).

3. A method according to either of claims 1 and 2 wherein a deaerated solvent medium is employed and the solution is subjected to inert gas purging before the container is sealed.

## Patentansprüche (Patentansprüche für folgende(n) Vertragsstaat(en): AT, BE, CH, DE, FR, GB, IT, LI, LU, NL, SE)

1. Pharmazeutische Formulierung einer stabilen, sterilen Lösung von Doxorubicin in einem verschlossenen Behälter, wobei die Lösung 1 bis 20 mg/ml eines physiologisch verträglichen Salzes von Doxorubicin und 0,1 bis 20 mg/ml Natriumacetonbisulfit in einem Lösungsmittelmedium enthält, bestehend aus 90-100% (v/v) Propylenglykol, 1,2-Dihydroxybutan oder 1,3-Dihydroxypropan und 0-10% (v/v) Wasser, und, falls erforderlich, eine Menge einer physiologisch verträglichen Säure oder Base, die zur Einstellung des pH-Werts der Lösung im Bereich von 3-6 erforderlich ist.

2. Formulierung nach Anspruch 1, worin ein entgastes Lösungsmittelmedium verwendet wird und in die Lösung vor dem Verschließen des Behälters Inertgas eingeblasen wird.

3. Formulierung nach Anspruch 1 oder 2, worin das Lösungsmittelmedium reines Propylenglykol ist.

4. Formulierung nach Anspruch 1 oder 2, worin das Lösungsmittelmedium reines 1,2-Dihydroxybutan ist.

5. Formulierung nach Anspruch 1 oder 2, worin das Lösungsmittelmedium reines 1,3-Dihydroxypropan ist.

6. Formulierung nach Anspruch 1 oder 2, worin das Lösungsmittelmedium Propylenglykol/Wasser (90:10 v/v) ist.

7. Formulierung nach Anspruch 1 oder 2, worin das Lösungsmittelmedium Propylenglykol/Wasser (95:5 v/v) ist.

8. Formulierung nach Anspruch 1 oder 2, worin das Lösungsmittelmedium 1,2-Dihydroxybutan/Wasser (90-95:5-10 v/v) ist.

9. Formulierung nach einem der Ansprüche 1 bis 8, worin das Lösungsmittelmedium aus 90-95% (v/v) Propylenglykol, 1,2-Dihydroxybutan oder 1,3-Dihydroxypropan und 5-10% (v/v) Wasser besteht.

10. Formulierung nach Anspruch 9, worin das Lösungsmittelmedium 1,3-Dihydroxypropan/Wasser (90-95:5-10 v/v) ist.

11. Verfahren zur Herstellung einer pharmazeutischen Formulierung einer stabilen, sterilen Lösung von Doxorubicin, wobei man bei diesem Verfahren 1 bis 20 mg/ml eines physiologisch verträglichen Salzes von Doxorubicin und 0,1 bis 20 mg/ml Natriumacetonbisulfit mit einem Lösungsmittelmedium mischt, bestehend aus 90-100% (v/v) Propylenglykol, 1,2-Dihydroxybutan oder 1,3-Dihydroxypropan und 0-10% (v/v) Wasser in Form einer Lösung, gegebenenfalls den pH der Lösung auf einen Wert im Bereich von 3-6 durch Zugabe einer physiologisch verträglichen Säure oder Base einstellt, die Lösung in einen Behälter gibt und den Behälter verschließt.

12. Verfahren zur Herstellung einer pharmazeutischen Formulierung nach Anspruch 11, worin die Wassermenge 5-10% (v/v) beträgt.

13. Verfahren nach Anspruch 11 oder Anspruch 12, worin ein entgastes Lösungsmittelmedium verwendet wird und in die Lösung vor dem Verschließen des Behälters Inertgas eingeblasen wird.

## Patentansprüche (Patentansprüche für folgende(n) Vertragsstaat(en): ES, GR)

1. Verfahren zur Herstellung einer pharmazeutischen Formulierung einer stabilen, sterilen Lösung von Doxorubicin, wobei man bei diesem Verfahren 1 bis 20 mg/ml eines physiologisch verträglichen Salzes von Doxorubicin und 0,1 bis 20 mg/ml Natriumacetonbisulfit mit einem Lösungsmittelmedium mischt, bestehend aus 90-100% (v/v) Propylenglykol, 1,2-Dihydroxybutan oder 1,3-Dihydroxypropan und 0-10% (v/v) Wasser in Form einer Lösung, gegebenenfalls den pH der Lösung auf einen Wert im Bereich von 3-6 durch Zugabe einer physiologisch verträglichen Säure oder Base einstellt, die Lösung in einen Behälter gibt und den Behälter verschließt.

2. Verfahren zur Herstellung einer pharmazeutischen Formulierung nach Anspruch 1, worin die Wassermenge 5-10% (v/v) beträgt.

3. Verfahren nach Anspruch 1 oder Anspruch 2, worin ein entgastes Lösungsmittelmedium verwendet wird und in die Lösung vor dem Verschließen des Behälters Inertgas eingeblasen wird.

## Revendications (Revendications pour l'(les) Etat(s) contractant(s) suivant(s): AT, BE, CH, DE, FR, GB, IT, LI, LU, NL, SE)

1. Une formulation pharmaceutique d'une solution stable, stérile de doxorubicine dans un container scellé, ladite solution contenant de 1 à 20 mg/ml d'un sel physiologiquement acceptable de doxorubicine et de 0,1 à 20 mg/ml d'acétone bisulfite de sodium dans un milieu solvant consistant en 90 à 100% (volume/volume) de propylèneglycol, 1,2-dihydroxybutane ou 1,3-dihydroxypropane, et de 0 à 10% (volume/volume) d'eau, et, si cela est nécessaire, une quantité d'un acide ou d'une base physiologiquement acceptable nécessaire pour ajuster le pH de la solution dans l'intervalle de 3 à 6.

2. Une formulation selon la revendication 1, dans laquelle un milieu solvant désaéré est utilisé et la solution est soumise à une purge avec un gaz inerte avant que le container ne soit scellé.

3. Une formulation selon la revendication 1 ou 2, dans laquelle le milieu solvant est du propylèneglycol pur.

4. Une formulation selon la revendication 1 ou 2, dans laquelle le milieu solvant est du 1,2-dihydroxybutane pur.

5. Une formulation selon la revendication 1 ou 2, dans laquelle le milieu solvant est du 1,3-dihydroxypropane pur.

6. Une formulation selon la revendication 1 ou 2, dans laquelle le milieu solvant est un mélange propylèneglycol/eau (90/10 volume/volume).

7. Une formulation selon la revendication 1 ou 2, dans laquelle le milieu solvant est un mélange propylèneglycol/eau (95/5, volume/volume).

8. Une formulation selon la revendication 1 ou 2, dans laquelle le milieu solvant est un mélange 1,2-dihdyroxybutane/eau (90 à 95/5 à 10, volume/volume).

9. Une formulation selon l'une quelconque des revendications 1 à 8, dans laquelle le milieu solvant consiste en 90 à 95% (volume/volume) de propylèneglycol, 1,2-dihydroxybutane ou 1,3-dihydroxypropane et de 5 à 10% (volume/volume) d'eau.

10. Une formulation selon la revendication 9, dans laquelle le milieu solvant est un mélange 1,3-dihydroxypropane/eau (90 à 95/5 à 10, volume/volume).

11. Un procédé de préparation d'une formulation pharmaceutique d'une solution de doxorubicine stable, stérile, ledit procédé comprenant les étapes de mélange de 1 à 20 mg/ml d'un sel physiologiquement acceptable de doxorubicine et de 0,1 à 20 mg/ml d'acétone bisulfite de sodium avec un milieu solvant consistant en 90 à 100% (volume/volume) de propylène glycol, 1,2-dihydroxybutane ou 1,3-dihydroxypropane et de 0 à 10% (volume/volume) d'eau pour former une solution, et l'étape d'ajustement, si cela est nécessaire, du pH de ladite solution dans l'intervalle de 3 à 6 par ajout d'un acide ou d'une base physiologiquement acceptable, la charge de ladite solution dans le container et le scellement dudit container.

12. Un procédé de préparation d'une formulation pharmaceutique selon la revendication 11, dans lequel la quantité d'eau est de 5 à 10% (volume/volume).

13. Un procédé selon la revendication 11 ou 12, dans lequel un milieu solvant désaéré est utilisé et la solution est soumise à une purge avec un gaz inerte avant que le container ne soit scellé.

## Revendications (Revendications pour l'(les) Etat(s) contractant(s) suivant(s): ES, GR)

1. Un procédé de préparation d'une formulation pharmaceutique d'une solution de doxorubicine stable, stérile, ledit procédé comprenant les étapes de mélange de 1 à 20 mg/ml d'un sel physiologiquement acceptable de doxorubicine et de 0,1 à 20 mg/ml d'acétone bisulfite de sodium avec un milieu solvant consistant en 90 à 100% (volume/volume) de propylène glycol, 1,2-dihydroxybutane ou 1,3-dihydroxypropane et de 0 à 10% (volume/volume) d'eau pour former une solution, et l'étape d'ajustement, si cela est nécessaire, du pH de ladite solution dans l'intervalle de 3 à 6 par ajout d'un acide ou d'une base physiologiquement acceptable, la charge de ladite solution dans le container et le scellement dudit container.

2. Un procédé de préparation d'une formulation pharmaceutique selon la revendication 1 , dans lequel la quantité d'eau est de 5 à 10% (volume/volume).

3. Un procédé selon la revendication 1 ou 2, dans lequel un milieu solvant désaére est utilisé et la solution est soumise à une purge avec un gaz inerte avant que le container ne soit scellé.
